# EUROPEAN PATENT SPECIFICATION

(11) **EP 2 320 749 B1**
(45) Date of publication and mention of the grant of the patent: **16.09.2015**
(21) Application number: 09781362.0
(22) Date of filing: 31.07.2009
(51) Int. Cl.: A23K 1/16, A23K 1/18, A23L 1/30, A23L 1/00, A61K 35/74

(54) **PRODUCTION OF BEADLETS COMPRISING PROBIOTIC COMPOUNDS**
HERSTELLUNG VON PROBIOTEN-ENTHALTENDEN KUGELCHEN
PRODUCTION DE PETITES BILLES CONTENANT DES PROBIOTIQUES

(30) Priority: 04.08.2008 EP 08161730
(43) Date of publication of application: 18.05.2011
(73) Proprietor: DSM IP Assets B.V., 6411 TE Heerlen (NL)
(72) Inventor: FUNDA, Elger, 79639 Grenzach-Wyhlen (DE); LEUTHARDT, Karin, 4144 Arlesheim (CH)
(74) Representative: Kurt, Manfred
(86) International application number: PCT/EP2009/059957
(87) International publication number: WO 2010/015580

(56) References cited:
- WO-A1-2004/062382
- WO-A1-2008/035332
- JP-A- 2 086 766
- US-A- 6 060 050
- US-A1- 2003 096 002
- US-A1- 2007 059 296
- US-A1- 2007 122 397
- KAILASAPATHY KAILA: "Microencapsulation of probiotic bacteria: technology and potential applications" CURRENT ISSUES IN INTESTINAL MICROBIOLOGY, HORIZON SCIENTIFIC PRESS, GB, vol. 3, no. 2, 1 September 2002 (2002-09-01), pages 39-48, XP009089483 ISSN: 1466-531X

## Description

The present invention relates to a process of production of beadlets comprising probiotic compounds in a matrix, which comprise at least one starch and/or starch derivative, to such beadlets and to the use of such specific beadlets.

The term "probiotic" generally refers to a non-pathogenic bacterium fed to animals, including birds, as a way to prevent colonization by pathogenic microorganisms, e.g. protozoa. Probiotics may also be defined as live, or livable, micro-organisms which beneficially affect the intestinal balance of healthy and normally functioning humans and animals.

Probiotics are delivered to farm animals for improving their intestinal microbial balance but also to human in the form of dairy-based foods containing intestinal species of lactobacilli and bifidobacteria.

Probiotics as live microorganisms are sensible to treat, store and formulate. Therefore the formulation of probiotics can be challenging.
The most common commercial formulation for probiotics are dairy products and probiotic fortified food (as well feed) products. However, tablets, capsules, powders and sachets containing the bacteria in freeze dried form are also available. Probiotics are also used in animal nutrition. Delivery systems for probiotics have been disclosed in e.g., WO 2008/035332 and US 6060050.
A very typical form of formulation of substances used in consumer products (such as for example food products) are powders. Powders can be produced by spray drying or freeze-drying processes. Freeze-drying is commonly used for drying of probiotics. Nevertheless this process is time-consuming and expensive. Moreover, viability of the bacteria may be negatively affected by the drying step.
Spray-drying is also used for drying of probiotics. This process is economically favorable but has certain disadvantages in the case of probiotics:
- Usually high temperatures (>60°C) are used in the production process. This has a negative effect on the activity of the probiotics.
- Using a low-temperature spray-drying processes with product temperatures <65°C usually leads to products with a relatively high content of residual water. Therefore, the storage stability of such powders is usually not sufficient.
- Powders are usually dusty and therefore their handling can be difficult.

Another well known form of preparations are beadlets. Beadlets provide superior handling properties in that they are not dusty and possess good flowablity characteristics. Beadlets are solely known for fat-soluble substances.
Beadlets (comprising fat-soluble substances) and their methods of productions are known from the prior art. These beadlets comprise fat-soluble (lipophilic, hydrophobic) substances. Such beadlets and their process for production are for example known from US2006/0115534 and US4670247. These beadlets usually have good storage stability, but the concentration of the fat-soluble substances in such beadlets is low. Usually the content is between 5 - 15 weight-% (wt-%), based on the total weight of the beadlet. Another disadvantage is that the production of such beadlets requires an emulsification or dispersion step to distribute the water-insoluble active in the aqueous matrix phase. Therefore, either the matrix material needs to have emulsifying properties or an additional emulsifier is required.

The goal of the present invention was to find a process for producing formulations of probiotics, which
- allows producing formulations with a high amount of probiotic bacteria,
- produces storage stable formulations, and
- is a simple process.

Surprisingly, it has been found that the use of the powder catch process allows producing such formulations, which are in the form of beadlets having the above mentioned advantages. The beadlets comprise at least one probiotic compound and at least one starch and/or at least one starch derivative as a matrix material. Furthermore, these beadlets are coated with a layer of the powder catch medium.

Therefore, the present invention relates to beadlets defined later, which comprise at least one probiotic compound, and may be produced by a process comprising
(a) forming an aqueous solution of
   (i) at least one probiotic compound and
   (ii) at least one starch and/or at least one starch derivative,
(b) converting the solution into a dry powder by spray drying into a collecting powder.

The principle of such a process is known from the prior art. It can be found for example in US6444227 or WO04062382. These references are hereby incorporated by reference.

Probiotic compounds are dietary supplements containing potentially beneficial bacteria or yeasts. According to the currently adopted definition by FAO/WHO, probiotics are: '*Live microorganisms which when administered in adequate amounts confer a health benefit on the host*'. Lactic acid bacteria (LAB) are the most common type of microbes used. LAB have been used in the food industry for many years, because they are able to convert sugars (including lactose) and other carbohydrates into lactic acid. This not only provides the characteristic sour taste of fermented dairy foods such as yogurt, but also by lowering the pH may create fewer opportunities for spoilage organisms to grow, hence creating possible health benefits on preventing gastrointestinal infections. Strains of the genera Lactobacillus and Bifidobacterium are the most widely used probiotic bacteria. Propionibacteria and other bacteria are also used as probiotics.
Probiotic bacterial cultures are intended to assist the body's naturally occurring gut flora, an ecology of microbes, to re-establish themselves. In animal nutrition, probiotics are intended to have beneficial effects on animal health. In the case of dairy cows, probiotics are intended to increase milk production and milk quality.
Probiotic bacterial cultures are for example Debaromyces, Candida, Pichia and Torulopsis, moulds such as Aspergillus, Rhizopus, Mucor, and Penicillium and Torulopsis and bacteria such as the genera Bifidobacterium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus and Lactobacillus. Specific examples of suitable probiotic micro-organisms (probiotic compounds) are: Saccharomyces cereviseae, Bacillus coagulans, Bacillus licheniformis, Bacillus subtilis, Bifidobacterium bifidum, Bifidobacterium infantis, Bifidobacterium longum, Enterococcus faecium, Enterococcus faecalis, Lactobacillus acidophilus, Lactobacillus alimentarius, Laciobacillus casei subsp. casei, Lactobacillus casei Shirota, Lactobacillus curvatus, Lactobacillus delbruckii subsp. lactis, Lactobacillus farciminus, Lactobacillus gasseri, Lactobacillus helveticus, Lactobacillus johnsonii, Lactobacillus reuteri, Lactobacillus rhamnosus (Lactobacillus GG), Lactobacillus sake, Lactococcus lactis, Micrococcus varians, Pediococcus acidilactici, Pediococcus pentosaceus, Pediococcus acidilactici, Pediococcus halophilus, Streptococcus faecalis, Streptococcus thermophilus, Staphylococcus carnosus, and Staphylococcus xylosus.
Preferred probiotic compounds used in functional food are lactic acid bacteria, mainly lactobacilli. Lactobacilli are non-pathogenic micro-organisms, colonizing the human intestinal and urogenital tract from early childhood to old age. Nowadays, several commercial probiotic lactobacilli are successfully used, among which Lactobacillus rhamnosus is one of the best known. Several strains of Lactobacillus fermentum are used for correction and stabilization of intestinal micro-flora in case of dysbacterioses and urogenital infections with different ethiologies.

Preferred examples of probiotics used as feed additive for farm animals are strains from the genera of Bacillus, Lactobacillus, Pediococcus and Propionibacterium.
A preferred strain of Bacillus is a strain of Bacillus licheniformis, preferably selected from the following strains of Bacillus licheniformis: ATCC 14580 (=NCIB 9375), NCIMB 6346 (=DSM 8785), NCTC 1024, NCTC 1025, NCTC 2120, NCTC 7589, NCTC 9932, ATCC 21424, NCIMB 10689, and ATCC 53757. A preferred subgroup includes Bacillus licheniformis ATCC 14580 (=NCIB 9375), and Bacillus licheniformis NCIMB 6346 (=DSM 8785).
A preferred strain of Lactobacillus is a strain of Lactobacillus reuteii, Lactobacillus acidophilus, Lactobacillus animalis, Lactobacillus ruminis, Lactobacillus johnsonii, Lactobacillus casei, Lactobacillus paracasei, Lactobacillus rhamnosus, and Lactobacillus fermentum. In a preferred embodiment the probiotic strain is selected from the group consisting of Lactobacillus reuteri (NCC2581; CNCM I-2448), Lactobacillus reuteri (NCC2592; CNCM I-2450), Lactobacillus rhamnosus (NCC2583; CNCM I-2449), Lactobacillus reuteri (NCC2603; CNCM I-2451), Lactobacillus reuteri (NCC2613; CNCM I-2452), and Lactobacillus acidophilus (NCC2628; CNCM I-2453).

More preferred is a process wherein the microorganism is of the genus Propionibacterium (P.) and more preferably P. acidipropionici and P. jensenii. Preferred strains of bacteria include P. acidipropionici and P. jensenii strains P169, P170, P179, P195, and P261, especially strain P169. The strains P 169 and P170 are available from the microorganism collection of the American Type Culture Collection (ATCC), 10801 University Blvd., Manassas, VA.,20110, under accession numbers ATCC PTA-5271 and ATCC PTA-5272, respectively, which have been deposited on June 18, 2003.

It is at present contemplated that the probiotic strain is administered in one or more of the following amounts (dosage ranges): 10 E2-14, 10 E4-12, 10 E6-10, 10 E7-9, preferably 10 E8 CFU/g of final feed (the designation E meaning exponent, viz., e.g., 10 E2-14 means 10^2-10^14).

CFU (Colony Forming Units) is defined as one or more, sometimes many, bacterial cells that grow into a visible colony on a Petri dish containing appropriate agar for the microorganism being tested. One colony is one CFU. For instance, 1 CFU may contain 20 bacterial cells or just one cell.

The invention is defined by the claims and relate to beadlets which comprise :
(i) 30 wt-% to 80 wt-%, preferably 40 wt-% to 70 wt-%, based on the total weight of the beadlets, of at least one probiotic compound chosen from the group consisting of Bacillus, Lactobacillus, Pediococcus and Propionibacterium, and
(ii) 15 wt-% - 65 wt-%, preferably 25 to 50 wt-%, based on the total weight of the beadlets, of at least one starch and/or starch derivates chosen from the group consisting of corn starch, sorghum starch, wheat starch, rice starch, tapioca starch, arrowroot starch, sago starch, potato starch, quinoa starch and amaranth starch, pregelatinised starches, acidic modified starches, oxidized starches, cross-linked starches, starch esters, starch ethers, dextrins and cationic starches, and
(iii) 5 wt-% to 50 wt-%, preferably 5 wt-% to 20 wt-%, based on the total weight of the beadlets, of powder coating chosen from the group consisting of starches, silicate and phosphate compounds,
wherein the size of the beadlets is 250 µm to 850 µm.

The invention also relates to the:
Use of the beadlets according to claims 1 to 2 in food products for humans, food products for animals dietary supplements for humans and dietary supplements for animals, as well as to the
Use of the beadlets in premixes for food products for humans, food products for animals dietary supplements for humans and dietary supplements for animals, and to
Food products for humans, food products for animals dietary supplements for humans and dietary supplements for animals comprising beadlets according to the invention , and to
Premix for food products for humans, food products for animals dietary supplements for humans and dietary supplements for animals comprising beadlets according to the invention.

In a preferred embodiment, the beadlets comprising the microorganism are fed to a ruminant, and the microorganism becomes established in the rumen. Preferably, the amount of the microorganism that is delivered to the ruminant is about 6 x 10⁹ CFU to about 6 x10¹²CFU/animal/day. This translates into approximately 1 x 10⁵ to1 x 10⁸ CFU/ml of rumen fluid for an averaged sized cow. In a more preferred embodiment, about 6 x 10¹¹ CFU/animal/day of the microorganism is delivered to the ruminant. In a preferred embodiment, the microorganism is fed to a ruminant such that the microorganism becomes established in the rumen at a level of about 1 x10⁵ CFU per ml of rumen fluid to about 1 x 10⁸ CFU per ml of rumen fluid.
The probiotic compounds are formulated in a beadlet by a matrix material, which comprises at least one starch and/or at least one starch derivative as defined above.

Starch having the chemical formula (C₆H₁₀O₅)ₙ is a polysaccharide carbohydrate consisting of a large number of glucose monosaccharide units joined together by glycosidic bonds.

All plant seeds and tubers contain starch. Starches are commonly extracted from plants, such as corn, sorghum, wheat, rice, tapioca, arrowroot, sago, potato, quinoa and amaranth.
Natural starches contain usually amylase and amylopectin molecules. The content of amylase in natural starches can vary from 0 wt-% (for example waxy corn starch and waxy rice starch) up to about 85 wt-% (High amylase corn starch). Normal starches contain about 25 wt-% of amylase. As a consequence thereof the content of amylopectin is between 15 wt-% and 100 wt-%.
It is also possible to use starch derivatives (modified starches) including hydrolyzed starches. The starches can be modified in various manners. It can be done physically and chemically.
Pregelatinised starches are examples of physically modified starches.
Acidic modified, oxidized, cross-linked, starch esters, starch ethers and cationic starches are examples of chemically modified starches. Important examples of such modified starches are octenyl succinic anhydride starches (OSA starches).
Dextrins like maltodextrin or yellow dextrin are examples of starch derivatives obtained by partial hydrolysis.
In a preferred process to produce the present invention the starches or starch derivatives are chosen from the group consisting of amylopectin, OSA starches, maltodextrin and pregelatinised starches.

A preferred process to produce the present invention is a powder catch process. Such a process is known from the prior art (for example from WO04062382). As a result of such a powder catch process the beadlets are covered by a layer of the powder.

Therefore, the beadlets produced according to this process are preferably covered by a layer of the powder catch medium. This layer (coating) is in the form of a powder coating. The powder catch medium is a compound (or a mixture of compounds), which is able to absorb moisture and to form a powder coating. Suitable powder catch media are i.e. starches, silicate or phosphate compounds. Preferred powder catch media are starches (such as i.e. corn starch), calcium silicate, calcium aluminum silicate and tri-calcium phosphate. Most preferred are starches, especially corn starch.
Beadlets are a well known form of formulation for fat-soluble substances. An important advantage of the generally spherical beadlets is that they are not dusty and that they posses excellent free flowing characteristics, which are very desirable for manufacturing and formulating operations.
The size of a beadlet is from 250µm to 850 µm. The size of a beadlet can be determined according to well known methods, such as (scanning) electron microscopy.
A suitable method to produce beadlets as disclosed and described above is for example described in WO 2004/062382.
The process to prepare the present invention surprisingly allows producing beadlets with a high amount of water-dispersible probiotic bacteria with good overall properties.

The beadlets comprise at least 30 wt-%, based on the total weight of the beadlets, of at least one probiotic compound.

The beadlets comprise up to 85 wt-%, based on the total weight of the beadlets, of at least one probiotic compound.

A preferred embodiment of the present invention relates to a process wherein the beadlets comprise up to 80 wt-%, based on the total weight of the beadlets, of at least one probiotic compound.

A preferred embodiment of the present invention relates to a process wherein the beadlets comprise at least 5 wt-%, based on the total weight of the beadlets, more preferred at least 20 wt-% of the powder coating layer.

The present invention relates to beadlets comprising:
(i) 5 wt-% - 80 wt-%, preferably 30 wt-% - 80 wt-%, more preferably 40 wt-% - 70 wt-%, based on the total weight of the beadlets, of at least one probiotic compound, and
(ii) 5 wt-% - 90 wt-%, preferably 25 wt-% - 50 wt-%, based on the total weight of the beadlets, of at least one starch and/or at least one starch derivative, and
(iii) 5 wt-% - 50 wt-%, preferably 5 wt-% - 20 wt-%, based on the total weight of the beadlets, of powder coating,
and wherein the size of the beadlets is of 250 µm to 850 µm.

An embodiment of the present invention relates to beadlets, wherein the starch component (ii), always comprise maltodextrin and at least one further starch and/or at least one further starch derivate.

The matrix of the beadlets of the present invention as described above can also comprise additional compounds. Such compounds can be any kind of auxiliaries used in the field of beadlet producing and/or food and feed technology. A preferred compound is sugar (sucrose).

Beadlets comprising a high amount (at least 30 wt-%) of probiotic compounds in a matrix comprising starch(es) and/or at least one starch derivative(s) are not known from the prior art.

The beadlets according to the present invention comprise
(i) 30 wt-% - 80 wt-%, preferably 40 wt-% - 70 wt-%, based on the total weight of the beadlets, of at least one probiotic compound chosen from the group consisting of Bacillus, Lactobacillus, Pediococcus and Propionibacterium, and
(ii) 15 wt-% - 65 wt-%, preferably 25 wt-% - 50 wt-%, based on the total weight of the beadlets, of at least one starch and/or at least one starch derivative chosen from the groups consisting of corn starch, sorghum starch, wheat starch, rice starch, tapioca starch, arrowroot starch, sago starch, potato starch, quinoa starch and amaranth starch, pregelatinised starches, acidic modified starches, oxidized starches, cross-linked starches, starch esters, starch ethers and cationic starches (preferred are starches with a high amount of amylopectin, OSA starches, (malto)dextrins and pregelatinised starches), and
(iii) 5 wt-% - 50 wt-%, preferably 5 wt-% - 20 wt-%, based on the total weight of the beadlets, of powder coating chosen from the group consisting of starches (preferably corn starch), silicate and phosphate compounds.

The size of the beadlets is of 250 µm to 850 µm.

In a more preferred embodiment of the present invention the starches or starch derivatives are chosen from the group consisting of amylopectin, OSA starches, maltodextrin and pregelatinised starches.

Most preferred beadlets according to the present invention comprise
(i) 40 wt-% - 70 wt-%, based on the total weight of the beadlets, of at least one probiotic compound chosen from the group consisting of acidipropionici and P. jensenii (preferably P. Acidipropionici and jensenii strains P169, P170, P179, P195, and P261, especially preferably strain P169), and
(ii) 25 wt-% - 50 wt-%, based on the total weight of the beadlets, of at least one starch and/or at least one starch derivative chosen from the groups consisting of corn starch, sorghum starch, wheat starch, rice starch, tapioca starch, arrowroot starch, sago starch, potato starch, quinoa starch and amaranth starch, pregelatinised starches, acidic modified starches, oxidized starches, cross-linked starches, starch esters, starch ethers, dextrins and cationic starches (preferred are starches with a high amount of amylopectin, OSA starches, (malto)dextrins and pregelatinised starches), and
(iii) 5 wt-% - 20 wt-%, based on the total weight of the beadlets, of powder coating chosen from the group consisting of starches (preferably corn starch), silicate and phosphate compounds.

A further embodiment according to the present invention relates to the use of the beadlets in food products (for humans and/or animals), dietary supplements as well in the production of food products and dietary supplements. Depending what kind of probiotic compounds and/or matrix material is used the food product is suitable for humans or animals. In some cases a food product could be consumed by humans and animals.

A preferred embodiment according to the present invention relates to the use of the beadlets in food for ruminants, especially for cows. Food products (for humans and/or animals) in the context of the present comprise liquid and solid food products as well as paste-like and or gel like. The food products comprise food for humans as well as for animals (especially ruminants, poultry and swine).

A dietary supplement, also known as food supplement or nutritional supplement, is a preparation intended to supply nutrients, such as vitamins, minerals, fatty acids or amino acids that are missing or are not consumed in sufficient quantity in a person's diet.
The food product can be in a ready-to-consume form that means a form, which is suitable to eat without further proceedings. But it is also possible that food product is a form, which needs further proceedings, like heating, dissolving, diluting, etc.
Suitable human food products can be drinks, soups, bars (cereal, chocolate), dairy products, etc.
Suitable animal food products (feed products) can be in any commonly used form. Therefore a further embodiment of the present invention relates to human and animal food products and to human and animal dietary supplements comprising beadlets as described above.
A preferred embodiment of the present invention relates to food products for ruminants (especially cows) comprising beadlets as described above.
The beadlets according to the present invention can also be used in premixes for food products (for humans and/or animals) and for dietary supplements (for humans and/or animals).
Premixes are a convenient usage form for the food producers but are a critical medium for probiotics due to pH, ionic strength and water activity values, which can negatively affect viability of probiotic bacteria. But the beadlets according to the present invention eliminate (or at least strongly minimize) such problems.
A preferred embodiment of the present invention relates to the use of the beadlets according to the present invention for the use in premixes for food for ruminants (especially cows).
A further embodiment of the present invention is a premix for food products (for humans and/or animals) and for dietary supplements (for humans and/or animals) comprising beadlets according to the present invention. A preferred embodiment of the present invention relates to a premix for food products for ruminants (especially cows).

Functional ingredients like vitamins and trace elements are often added to food or feed products as well as to premixes.

The following examples serve to illustrate the invention. The percentages are expressed in weight percentages and the temperatures are degrees Celsius, if not otherwise defined.

**Example 1 (comparative):** *Formulation of propionibacteria (P169) in a matrix comprising amylopectin* 1,5 g amylopectin were added to 25 ml of water. The mixture was heated and stirred until dissolution occurred. The pH of the solution was adjusted to 7.0. To this solution, 50 g of concentrated biomass of propionibacteria (20% dry matter) were added under stirring. About 75 g of the suspension was sprayed in a spraying pan in a bed of fluidized starch at about 5°C by means of a rotating spraying nozzle. The so-obtained beadlets were separated from excess starch by sieving and dried. There were obtained ca. 30 g of dry powder having an activity of 4,6E11 cfu/g.

### Example 2: Probiotic formulation with maltodextrin and OSA-starch as matrix

4.66 kg maltodextrin and 0.53 kg OSA-starch were dissolved in 35 kg concentrated biomass of propionibacteria (15% dry matter). The suspension was sprayed together with fluidized starch using a pilot-scale beadlet tower. The so-obtained beadlets were separated from excess starch and dried in an external fluid-bed. There were obtained ca. 13 kg of dry powder having an activity of 1.5E11 cfu/g.

### Example 3: Probiotic formulation with maltodextrin as matrix

5.17 kg maltodextrin were dissolved in 35 kg concentrated biomass of propionibacteria (15% dry matter). The suspension was sprayed together with fluidized starch using a pilot-scale beadlet tower. The so-obtained beadlets were separated from excess starch and dried in an external fluid-bed. There were obtained ca. 13 kg of dry powder having an activity of 9.6E10 cfu/g.

### Example 4 (comparative): Propionibacteria (P969) in a matrix comprising fish gelatine and sugar

40 g of fish gelatine and 20 g of sucrose were added to 80 ml of water. The mixture was stirred until dissolution occurred. The pH of the solution was adjusted to 7.0. To this solution, 60 g of concentrated biomass of propionibacteria (20% dry matter) were added under stirring. About 180 g of the suspension was sprayed in a spraying pan in a bed of fluidized starch at about 5°C by means of a rotating spraying nozzle. The so-obtained beadlets were separated from excess starch by sieving and dried. There were obtained ca. 120 g of dry powder having an activity of 8,2E10 cfu/g.

### Example 5: Propionibacteria (P169) in a matrix comprising gum arabic and maltodextrin

150 g concentrated biomass of propionibacteria (20% dry matter) were diluted with 50g water. 52g maltodextrin and 10g gum arabic were added. The mixture was stirred until dissolution occurred. About 240 g of the suspension was sprayed in a spraying pan in a bed of fluidized starch at about 5°C by means of a rotating spraying nozzle. The so-obtained beadlets were separated from excess starch by sieving and dried. There were obtained ca. 160 g of dry powder having a activity of 1,5E11 cfu/g.

### Example 6 (comparative): Propionibacteria (P169) in a matrix comprising polyethylene glycol

5g of polyethylene glycol PEG1000 were molten at 45°C and added to 50 g concentrated biomass of propionibacteria (20% dry matter) with stirring. 5g water was added. The suspension was sprayed in a spraying pan in a bed of fluidized starch at about 5°C by means of a rotating spraying nozzle. The so-obtained beadlets were separated from excess starch by sieving and dried. There were obtained ca. 24 g of dry powder having an activity of 4.1 E 11 cfu/g.

## Claims

1. Beadlets comprising
(i) 30 wt-% to 80 wt-%, preferably 40 wt-% to 70 wt-%, based on the total weight of the beadlets, of at least one probiotic compound chosen from the group consisting of Bacillus, Lactobacillus, Pediococcus and Propionibacterium, and
(ii) 15 wt-% - 65 wt-%, preferably 25 to 50 wt-%, based on the total weight of the beadlets, of at least one starch and/or starch derivative chosen from the group consisting of corn starch, sorghum starch, wheat starch, rice starch, tapioca starch, arrowroot starch, sago starch, potato starch, quinoa starch and amaranth starch, pregelatinised starches, acid modified starches, oxidized starches, cross-linked starches, starch esters, starch ethers, dextrins and cationic starches, and
(iii) 5 wt-% to 50 wt-%, preferably 5 wt-% to 20 wt-%, based on the total weight of the beadlets, of powder coating chosen from the group consisting of starches, silicate and phosphate compounds,
wherein the size of the beadlets is 250 µm to 850 µm..

2. Beadlets according to claims 1, wherein the probiotic compounds is chosen from the group consisting of P. Acidipropionici and jensenii strains P169, and P170, preferably P169.

3. Use of the beadlets according to claims 1 to 2 in food products for humans, food products for animals dietary supplements for humans and dietary supplements for animals.

4. Use of the beadlets according to claims 1 to 2 in premixes for food products for humans, food products for animals dietary supplements for humans and dietary supplements for animals.

5. Food products for humans, food products for animals dietary supplements for humans and dietary supplements for animals comprising beadlets according to claims 1 to 2.

6. Premix for food products for humans, food products for animals dietary supplements for humans and dietary supplements for animals comprising beadlets according to claims 1 to 2.

## Patentansprüche

1. Kügelchen, umfassend
(i) 30 Gew.-% bis 80 Gew.-%, vorzugweise 40 Gew.-% bis 70 Gew.-%, in Bezug auf das Gesamtgewicht der Kügelchen an mindestens einer probiotischen Verbindung, ausgewählt aus der Gruppe bestehend aus Bacillus, Lactobacillus, Pediococcus und Propionibacterium, und
(ii) 15 Gew.-% bis 65 Gew.-%, vorzugweise 25 bis 50 Gew.-%, in Bezug auf das Gesamtgewicht der Kügelchen, an mindestens einer Stärke und/oder einem Stärkederivat, ausgewählt aus der Gruppe bestehend aus Maisstärke, Sorghumstärke, Weizenstärke, Reisstärke, Cassavastärke, Pfeilwurzstärke, Sagostärke, Kartoffelstärke, Quinoastärke und Amaranthstärke, vorverkleisterten Stärken, säuremodifizierten Stärken, oxidierten Stärken, vernetzten Stärken, Stärkeestern, Stärkeethern, Dextrinen und kationischen Stärken, sowie
(iii) 5 Gew.-% bis 50 Gew.-%, vorzugweise 5 Gew.-% bis 20 Gew.-%, in Bezug auf das Gesamtgewicht der Kügelchen, an Pulverbeschichtung, ausgewählt aus der Gruppe bestehend aus Stärken, Silikat- und Phosphatverbindungen,
wobei die Größe der Kügelchen 250 µm bis 850 µm beträgt.

2. Kügelchen nach Anspruch 1, wobei die probiotische Verbindung aus der Gruppe bestehend aus den P. acidipropionici- und jensenii-Stämmen P169 und P170, vorzugsweise P169, ausgewählt ist.

3. Verwendung der Kügelchen nach den Ansprüchen 1 und 2 in Nahrungsmittelprodukten für den Menschen, Futtermittelprodukten für Tiere, Nahrungsergänzungsmitteln für den Menschen und Futterergänzungsmitteln für Tiere.

4. Verwendung der Kügelchen nach den Ansprüchen 1 bis 2 in Vormischungen für Nahrungsmittelprodukte für den Menschen, Futtermittelprodukte für Tiere, Nahrungsergänzungsmittel für den Menschen und Futterergänzungsmittel für Tiere.

5. Nahrungsmittelprodukte für den Menschen, Futtermittelprodukte für Tiere, Nahrungsergänzungsmittel für den Menschen und Futterergänzungsmittel für Tiere, umfassend Kügelchen nach den Ansprüchen 1 bis 2.

6. Vormischungen für Nahrungsmittelprodukte für den Menschen, Futtermittelprodukte für Tiere, Nahrungsergänzungsmittel für den Menschen und Futterergänzungsmittel für Tiere, umfassend Kügelchen nach den Ansprüchen 1 bis 2.

## Revendications

1. Granules comprenant
(i) 30 % en poids à 80 % en poids, de préférence 40 % en poids à 70 % en poids, sur la base du poids total des granules, d'au moins un composé probiotique choisi dans le groupe constitué par *Bacilles*, *Lactobacillus*, *Pediococcus* et *Propionibacterium* et
(ii) 15 % en poids à 65 % en poids, de préférence 25 à 50 % en poids, sur la base du poids total des granules, d'au moins un amidon et/ou dérivé d'amidon choisi dans le groupe constitué par l'amidon de maïs, l'amidon de sorgho, l'amidon de blé, l'amidon de riz, la fécule de manioc, la fécule de Maranta, la fécule de sagou, la fécule de pomme de terre, l'amidon de quinoa et l'amidon d'amarante, les amidons prégélatinisés, les amidons modifiés par un acide, les amidons oxydés, les amidons réticulés, les esters d'amidon, les éthers d'amidon, les dextrines et les amidons cationiques et
(iii) 5 % en poids à 50 % en poids, de préférence 5 % en poids à 20 % en poids, sur la base du poids total des granules, de revêtement en poudre choisi dans le groupe constitué par les amidons, les composés silicates et les composés phosphates,
la taille des granules étant de 250 µm à 850 µm.

2. Granules selon la revendication 1, dans lesquels les composés probiotiques sont choisis dans le groupe constitué par les souches P169 et P170, de préférence P169 de *P. acidipropionici* et *jensenii.*

3. Utilisation des granules selon les revendications 1 à 2 dans des produits alimentaires pour l'homme, des produits alimentaires pour animaux, des compléments alimentaires pour l'homme et des compléments alimentaires pour animaux.

4. Utilisation des granules selon les revendications 1 à 2 dans des prémélanges pour des produits alimentaires pour l'homme, des produits alimentaires pour animaux, des compléments alimentaires pour l'homme et des compléments alimentaires pour animaux.

5. Produits alimentaires pour l'homme, produits alimentaires pour animaux, compléments alimentaires pour l'homme et compléments alimentaires pour animaux comprenant des granules selon les revendications 1 à 2.

6. Prémélange pour produits alimentaires pour l'homme, produits alimentaires pour animaux, compléments alimentaires pour l'homme et compléments alimentaires pour animaux comprenant des granules selon les revendications 1 à 2.
